# EUROPEAN PATENT APPLICATION

(11) **EP 1 359 143 A1**
(43) Date of publication of application: **05.11.2003**
(21) Application number: 02710482.7
(22) Date of filing: 04.02.2002
(51) Int. Cl.: C07C 403/22, C07C 317/14, C07C 315/00

(54) **NOVEL SULFONE DERIVATIVES AND PROCESS FOR PRODUCING THESE**

(30) Priority: 06.02.2001 JP 2001029312; 26.02.2001 JP 2001049820; 02.03.2001 JP 2001057924
(71) Applicant: SUMITOMO CHEMICAL COMPANY LIMITED, Osaka-shi, Osaka 541-0041 (JP)
(72) Inventor: KIMURA, Kazutaka, Ibaraki-shi, Osaka 567-0845 (JP); TAKAHASHI, Toshiya, Ibaraki-shi, Osaka 567-0826 (JP); SEKO, Shinzo, Toyonaka-shi, Osaka 560-0003 (JP)
(74) Representative: Henkel, Feiler, Hänzel
(86) International application number: JP0200869
(87) International publication number: WO02062752

(57) **Abstract**

There are provided production processes for cyclic sulfone compounds of formula (2) or (4): 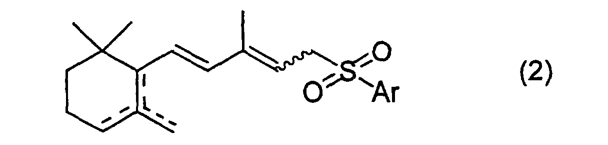 or 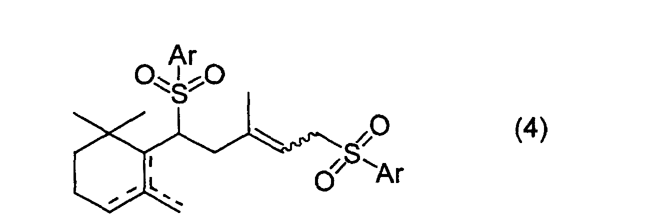 wherein the dashed lines represent that a double bond is present at one of three positions indicated; Ar and the wavy line are as defined below, characterized in that compounds of formula (1) or (3): 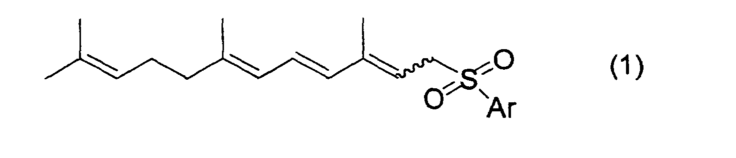 or 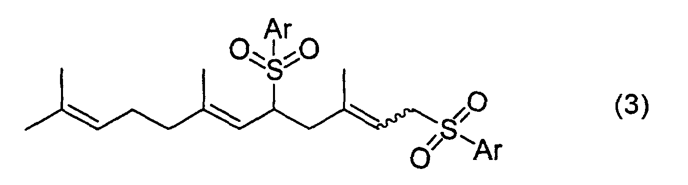 wherein Ar is aryl optionally having a substituent(s) and the wavy line represents either one of E/Z geometrical isomers or their mixture, is subjected to cyclization in the presence of an acid catalyst. These processes are excellent processes as the production processes for retinol from the viewpoints of raw material costs, intermediate purification, the number of steps, and the like.

## Description

### Technical Field

The present invention relates to disulfone derivatives and conjugated triene derivatives, which are useful as the intermediates of pharmaceuticals, feed additives, and food additives, *e.g.,* as the intermediates of retinol derivatives and carotenoids, as well as their production processes.

### Background Art

There have not been known so far the linear disulfone compounds of the following formula (11) and the conjugated triene compounds of the following formula (10).

The present inventors have found sulfone derivatives as the important intermediates of retinol by the coupling reaction of cyclic sulfones and allyl halides derived from C10 alcohols *(e.g.,* geraniol), as disclosed in JP-A 11-222479.

### Purpose of the Invention

Regarding the production process for retinol, there has been a great demand for the development of a further excellent production process from the viewpoints of raw material costs, intermediate purification, the number of steps, and the like.

The present invention has been made for the purpose of providing such a production process.

### Summary of the Invention

The present inventors have extensively studied to attain the above purpose. As a result, they have found linear disulfone compounds of the following formula (3), which can derived from sulfone compounds of the following formula (6) and allyl halide derivatives of the following formula (7) or (8); and that cyclic sulfone derivatives of the following formulas (4) and (2) as the useful intermediates of retinol derivatives and carotenoids can be produced by reacting conjugated triene compounds of formula (1), which can be derived in one step from linear disulfone compounds (3), with an acid catalyst, thereby completing the present invention.

Thus, the present invention provides:
[1] a process for the production of a cyclic sulfone compound of formula (2): 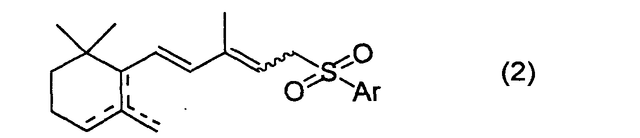 wherein the dashed lines represent that a double bond is present in one of three positions indicated; Ar and the wavy line are as defined below, characterized in that a conjugated triene compound of formula (1): 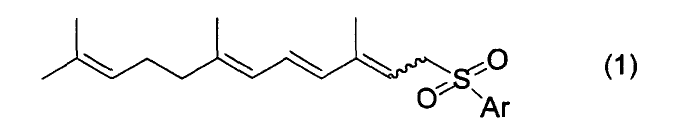 wherein Ar is aryl optionally having a substituent(s) and the wavy line represents either one of E/Z geometrical isomers or their mixture, is subjected to cyclization in the presence of an acid catalyst;
[2] a process for the production of a conjugated triene derivative of formula (1), characterized in that a linear disulfone compound of formula (3): 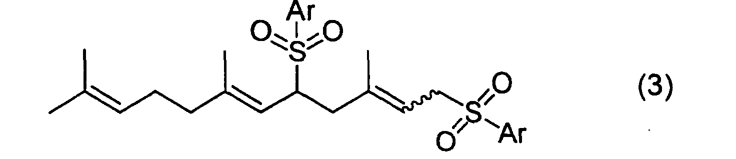 wherein Ar and the wavy line are as defined above, is reacted with a base;
[3] a process for the production of a cyclic disulfone compound of formula (4): 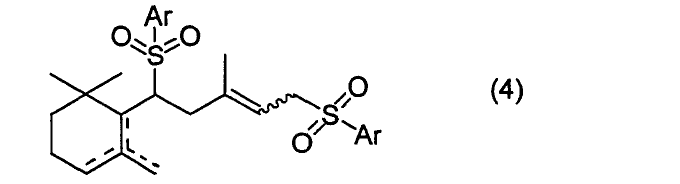 wherein Ar, the dashed lines, and the wavy line are as defined above, characterized in that a linear disulfone compound of formula (3) is subjected to cyclization in the presence of an acid catalyst;
[4] a process of the production of a linear disulfone compound of formula (3), characterized in that a linear sulfone compound of formula (5): 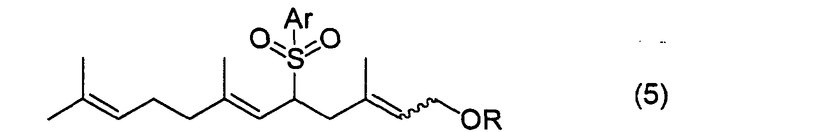 wherein R is a protective group for the hydroxyl group; Ar and the wavy line are as defined above, is subjected to sulfonation;
[5] a process for the production of a linear disulfone compound of formula (3), characterized in that a sulfone compound of formula (6): 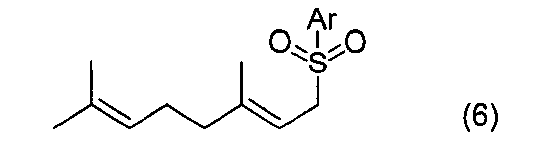 wherein Ar is as defined above, is reacted with an allyl halide of formula (7): 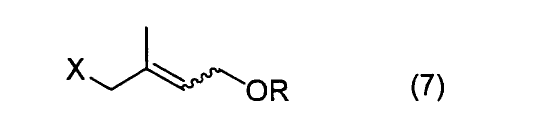 wherein X is halogen; R and the wavy line are as defined above, in the presence of a basic compound to give a linear sulfone compound of formula (5), and the resulting linear sulfone (5) is reacted with an arylsulfinate of formula (9): ArSO₂M wherein Ar is as defined above and M is an alkali metal;
[6] a process for producing a linear disulfone compound of formula (3), characterized in that a sulfone compound of formula (6) is reacted with a halosulfone compound of formula (8): 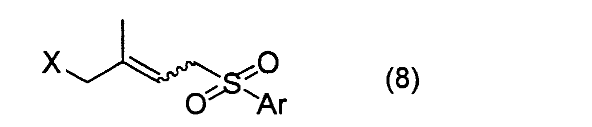 wherein X, Ar, and the wavy line are as defined above, in the presence of a basic compound;
[7] a conjugated triene compound of formula (10): 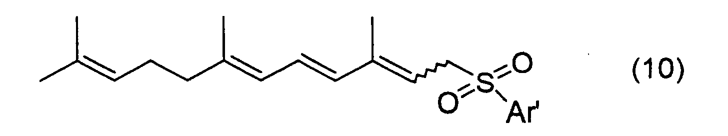 wherein Ar' is phenyl having a substituent(s) and the wavy line is as defined above; and
[8] a linear disulfone compound of formula (11): 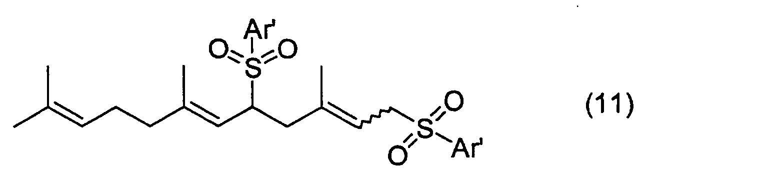
wherein Ar' and the wavy line are as defined above.

### Description of Preferred Embodiments of the Invention

The R in the compounds of formulas (5) and (7) represents a protective group for the hydroxy group, and the protective group of the hydroxy group may include acyl such as formyl, acetyl, ethoxyacetyl, fluoroacetyl, di-fluoroacetyl, trifluoroacetyl, chloroacetyl, dichloroacetyl, trichloroacetyl, bromoacetyl, dibromoacetyl, tribromoacetyl, propionyl, 2-chloropropionyl, 3-chloropropionyl, butyryl, 2-chlorobutyryl, 3-chlorobutyryl, 4-chlorobutyryl, 2-methylbutyryl, 2-ethylbutyryl, valeryl, 2-methylvaleryl, 4-methylvaleryl, hexanoyl, isobutyryl, isovaleryl, pivaloyl, benzoyl, o-chlorobenzoyl, m-chlorobenzoyl, p-chlorobenzoyl, o-hydroxybenzoyl, m-hydroxybenzoyl, p-hydroxybenzoyl, o-acetoxybenzoyl, o-methoxybenzoyl, m-methoxybenzoyl, p-methoxybenzoyl, and p-nitrobenzoyl; sillyl such as trimethylsillyl, triethylsillyl, t-butyldimethylsillyl, and t-butyldiphenylsillyl; alkoxyalkyl such as tetrahydropyranyl, methoxymethyl, methoxyethoxymethyl, and 1-ethoxyethyl; benzyl; p-methoxybenzyl; t-butyl; trityl; 2,2,2-trichloroethoxycarbonyl; and allyloxycarbonyl. Usually preferred is acyl, and acetyl is more preferably used.

The Ar in the compounds of formulas (1), (2), (3), (4), (5), (6), and (8) represents aryl optionally having a substituent(s), and the aryl may include phenyl and naphthyl. The substituent(s) may include C₁-C₅ straight chain or branched alkyl, C₁-C₅ straight chain or branched alkoxy, halogen *(e.g,* fluorine, chlorine, bromine, iodine), and nitro.

The C₁-C₅ alkyl may include methyl, ethyl, n-propyl, i-propyl, n-butyl, s-butyl, t-butyl, n-pentyl, and neo-pentyl. The C₁-C₅ alkoxy may include methoxy, ethoxy, n-propoxy, i-propoxy, n-butoxy, s-butoxy, t-butoxy, n-pentyl-oxy, and neo-pentyloxy.

Specific examples of the substituent Ar may include phenyl, naphthyl, o-tolyl, m-tolyl, p-tolyl, o-methoxyphenyl, m-methoxyphenyl, p-methoxyphenyl, o-chlorophenyl, m-chlorophenyl, p-chlorophenyl, o-bromophenyl, m-bromophenyl, p-bromophenyl, o-iodophenyl, m-iodophenyl, p-iodophenyl, o-fluorophenyl, m-fluorophenyl, p-fluorophenyl, o-nitrophenyl, m-nitrophenyl, and p-nitrophenyl. More preferred is tolyl.

The Ar' shown in formulas (10) and (11) represents phenyl having a substituent(s). The substituent(s) may include C₁-C₅ straight chain or branched alkyl, C₁-C₅ straight chain or branched alkoxy, halogen, and nitro. The C₁-C₅ alkyl, alkoxy, and halogen may include the same as described above for their specific examples.

Specific examples of the substituent Ar' may include o-tolyl, m-tolyl, p-tolyl, o-methoxyphenyl, m-methoxyphenyl, p-methoxyphenyl, o-chlorophenyl, m-chlorophenyl, p-chlorophenyl, o-bromophenyl, m-bromophenyl, p-bromophenyl, o-iodophenyl, m-iodophenyl, p-iodophenyl, o-fluorophenyl, m-fluorophenyl, p-fluorophenyl, o-nitrophenyl, m-nitrophenyl, and p-nitrophenyl. More preferred is tolyl.

The X in the allyl halide derivatives of formulas (7) and (8) represents halogen, specific examples of which are chlorine, bromine, iodine, and the like.

The sulfone compound (6) as the raw material used in the present invention can easily be produced by the process, for example, as described in J. Org. Chem. 39, 2135 (1974); the allyl halide compound (7), by the process as described in the specification of U.S. Patent No. 4,175,204; and the halosulfone compound (8), by the following scheme 1: 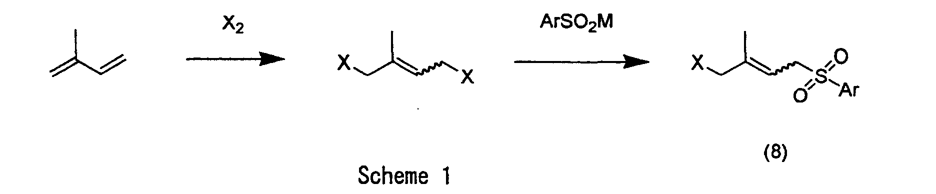 wherein M is an alkali metal; Ar, X, and the wavy line are as defined above.

The cyclic sulfone compound of formula (2) can be produced by the cyclization of the conjugated triene compound of formula (1) in the presence of an acid catalyst.

The cyclic disulfone compound of formula (4) can be produced by the cyclization of the linear disulfone compound of formula (3) in the presence of an acid catalyst.

The acid catalyst used in the above reactions may include protic acids, Lewis acids, and solid acids.

The protic acid may include sulfuric acid, acetic acid, hydrochloric acid, phosphoric acid, polyphosphoric acid, trifluoroacetic acid, perchloric acid, perbromic acid, periodic acid, benzenesulfonic acid, p-toluenesulfonic acid, methanesulfonic acid, and trifluoromethanesulfonic acid.

The Lewis acid may include halides of zinc, aluminum, zirconium, tin, copper, titanium, or boron, specific examples of which zinc chloride, zinc bromide, zinc iodide, aluminum chloride, zirconium chloride, stannous chloride, stannous bromide, stannous fluoride, cuprous chloride, cupric chloride, cuprous iodide, titanium tetrachloride, and boron trifluoride ether complex.

The solid acid may include zeolite, cation exchange resins, and sulfuric acid treated products of zirconia, titania, or alumina. A specific example of the zeolite is H-US-Y zeolite. The preferred cation exchange resin has sulfonic acid groups as functional groups. Specific examples are Amberlist 15DRY™, Amberlist 15WET™, Amberlist 16WET™, Amberlist 31WET™, Duolite C26TRH™, Duolite C255LFH™, Duolite SC100™, Duolite SC200™, Duolite SC300™, Duolite SC400™, Duolite SC500™, Duolite SC600™, Nafion NE417™, Nafion NR50™, and Nafion SAC-13™.

In particular, Amberlist 15DRY™ is preferably used.

The above acid catalysts may be used alone or as a mixture of two or more.

The amount of acid catalyst used is not particularly limited.

When the acid catalyst is an aqueous solution, a phase transfer catalyst may be add to further proceed the reaction.

The phase transfer catalyst used in the above reaction may include quaternary ammonium salts, quaternary phosphonium salts, and sulfonium salts.

The quaternary ammonium salts may include tetramethylammonium chloride, tetraethylammonium chloride, tetrapropylammonium chloride, tetrabutylammonium chloride, tetrapentylammonium chloride, tetrahexylammonium chloride, tetraheptylammonium chloride, tetraoctylammonium chloride, tetrahexadecylammonium chloride, tetraoctadecylammonium chloride, benzyltrimethylammonium chloride, benzyltriethylammonium chloride, benzyltributylammonium chloride, 1-methylpyridinium chloride, 1-hexadecylpyridinium chloride, 1,4-dimethylpyridinium chloride, tetramethyl-2-butylammonium chloride, trimethylcylopropylammonium chloride, tetramethylammonium bromide, tetraethylammonium bromide, tetrapropylammonium bromide, tetrabutylammonium bromide, tetrapentylammonium bromide, tetrahexylammonium bromide, tetraheptylammonium bromide, tetraoctylammonium bromide, tetrahexadecylammonium bromide, tetraoctadecylammonium bromide, benzyltrimethylammonium bromide, benzyltriethylammonium bromide, benzyltributylammonium bromide, 1-methylpyridinium bromide, 1-hexadecylpyridinium bromide, 1,4-dimethylpyridinium bromide, tetramethyl-2-butylammonium bromide, trimethylcylopropylammonium bromide, tetramethylammonium iodide, tetrabutylammonium iodide, tetraoctylammonium iodide, t-butylethyldimethylammonium iodide, tetradecyltrimethylammonium iodide, hexadecyltrimethylammonium iodide, octadecyltrimethylammonium iodide, benzyltriethylammonium iodide, benzyltributylammonium iodide, tetramethylammonium hydrogensulfate, tetrabutylammonium hydrogensulfate, tetraoctylammonium hydrogensulfate, t-butylethyldimethylammonium hydrogensulfate, tetradecyltrimethylammonium hydrogensulfate, hexadecyltrimethylammonium hydrogensulfate, octadecyltrimethylammonium hydrogensulfate, benzyltrimethylammonium hydrogensulfate, benzyltriethylammonium hydrogensulfate, benzyltributylammonium hydrogensulfate, and tetra-n-butyl-ammonium hydrogensulfate.

The quaternary phosphonium salt may include tributylmethylphosphonium chloride, triethylmethylphosphonium chloride, methyltriphenoxyphosphonium chloride, butyltriphenylhosphonium chloride, tetrabutylphosphonium chloride, benzyltriphenylphosphonium chloride, hexadecyltrimethylphosphonium chloride, hexadecyltributylphosphonium chloride, hexadecyldimethylethylphosphonium chloride, tetraphenylphosphonium chloride, tributylmethylphosphonium bromide, triethylmethylphosphonium bromide, methyltriphenoxyphosphonium bromide, butyltriphenylphosphonium bromide, tetrabutylphosphonium bromide, benzyltriphenylphosphonium bromide, hexadecyltrimethylphosphonium bromide, hexadecyltributylphosphonium bromide, hexadecyldimethylethylphosphonium bromide, tetraphenylphosphonium bromide, tributylmethylphosphonium iodide, triethylmethylphosphonium iodide, methyltriphenoxyphosphonium iodide, butyltriphenylphosphonium iodide, tetrabutylphosphonium iodide, benzyltriphenylphosphonium iodide, and hexadecyltrimethylphosphonium iodide.

The sulfonium salt may include dibutylmethylsulfonium chloride, trimethylsulfonium chloride, triethylsulfonium chloride, dibutylmethylsulfonium bromide, trimethylsulfonium bromide, triethylsulfonium bromide, dibutylmethylsulfonium iodide, trimethylsulfonium iodide, and triethylsulfonium iodide.

In particular, quaternary ammonium salts are preferably used.

The amount of such a phase transfer catalyst used is usually about 0.01 to 0.2 mole, preferably about 0.02 to 0.1 mole, relative to 1 mole of the linear sulfone derivative (1) or (3).

The above reaction may be carried out without any solvent or using a solvent when an acid is a liquid, or may preferably be carried out using a solvent when an acid is a solid.

When a solvent is used, the solvent used may include water; ether solvents such as diethyl ether, tetrahydrofuran, 1,4-dioxane, dimethoxyethane, and anisole; aprotic polar solvents such as acetonitrile, N,N-dimethyl-formamide, hexamethylphosphoric triamide, sulfolane, 1,3-dimethyl-2-imidazolidinone, and 1-methyl-2-pyrrolidinone; hydrocarbon solvents such as n-hexane, cyclohexane, n-pentane, benzene, toluene, and xylene; and halogen solvents such as dichloromethane, chloroform, and carbon tetrachloride. These may be used alone or as a mixture of two or more.

The reaction temperature can freely be selected usually in the range of ―78°C to the boiling point of a solvent, preferably in the range of about 20°C to 60°C. After the reaction, the cyclic sulfone compound (2) or the cyclic disulfone compound (4) can be obtained by the ordinary post-treatment, for example, operations including filtration, water washing, extraction, crystallization, and various chromatographies.

The conjugated triene derivatives of formula (1) can be obtained by reacting the linear disulfone compound of formula (3) with a base.

The base used in the reaction may include hydroxides of alkali metals, hydrides of alkali metals, alkoxides of alkali metals, and amides of alkali metals. Specific examples of the hydroxides of alkali metals are lithium hydroxide, sodium hydroxide, and potassium hydroxide; specific examples of the hydrides of alkali metals are sodium hydride and potassium hydride; specific examples of the alkoxides of alkali metals are sodium t-butoxide, potassium t-butoxide, potassium t-butoxide, sodium methoxide, and potassium methoxide; and specific examples of the amides of alkali metals are sodium amide and potassium amide. In particular, the hydroxides of alkali metals are preferably used. With regard to the form, those in fine powder form are more preferred. The amount for their use is usually in the range of about 2 to 20 moles, preferably in the range of about 3 to 15 moles, relative to 1 mole of the disulfone derivative of formula (1).

The above reaction can proceed only with the hydroxide of an alkali metal, but a lower alcohol or a phase transfer catalyst may be added to further proceed the reaction.

The lower alcohol used in the above reaction may include C₁-C₅ alkyl alcohols such as methanol, ethanol, i-propylalcohol, s-butylalcohol, and t-butylalcohol. The amount for their use is usually about 0.5 to 3 moles, relative to 1 mole of the linear disulfone compound of formula (3).

The phase transfer catalyst used in the above reaction may include the same as described above. In particular, quaternary ammonium salts are preferably used.

The amount of such a phase transfer catalyst used is usually about 0.01 to 0.2 mole, preferably about 0.02 to 0.1 mole, relative to 1 mole of the linear disulfone compound (3).

In the above reaction, an organic solvent is usually used. The solvent may include ether solvents such as diethyl ether, tetrahydrofuran, dimethoxyethane, dioxane, and anisole; hydrocarbon solvents such as n-hexane, cyclohexane, n-pentane, toluene, and xylene; and aprotic polar solvents such as acetonitrile, N,N-dimethylformamide, hexamethylphosphoric triamide, sulfolane, 1,3-dimethyl-2-imidazolidinone, and 1 -methyl-2-pyrrolidinone. These may be used alone or as a mixture of two or more. Preferably, hydrocarbon solvents such as toluene and hexane are used.

The reaction temperature is usually in the range of-30°C to the boiling point of a solvent used, preferably about 0°C to 70°C.

After the reaction, the conjugated triene compound (1) can be produced by the ordinary post-treatment, for example, operations including water washing, extraction, crystallization, and various chromatographies.

The linear disulfone compound of formula (3) can be produced by subjecting the linear sulfone compound of formula (5) to sulfonation.

In the above reaction, for example, arylsulfinates of formula (9):

ArSO₂M (9)

wherein Ar is as defined above and M is an alkali metal, are used.

The M in the arylsulfinates of formula (9) represents an alkali metal, specific examples of which are lithium, sodium, and potassium.

The arylsulfinates of formula (9) may include lithium p-toluenesulfinate, sodium p-toluenesulfinate, potassium p-toluenesulfinate, lithium p-chlorophenylsulfinate, sodium p-chlorophenylsulfinate, potassium p-chlorophenylsulfinate, lithium p-bromophenylsulfinate, sodium p-bromophenylsulfinate, potassium p-bromophenylsulfinate, lithium p-iodophenylsulfinate, sodium p-iodophenylsulfinate, potassium p-iodophenylsulfinate, lithium p-nitrophenylsulfinate, sodium p-nitrophenylsulfinate, and potassium p-nitrophenylsulfinate. Preferably, sodium p-toluenesulfinate and potassium p-toluenesulfinate are used. These may be hydrates. The amount for their use is usually about 1 to 3 moles, relative to 1 mole of the linear sulfone compound (5).

For the above reaction, palladium catalysts may be used and may include tetrakistriphenylphosiphine palladium, allyl chloride palladium dimer, palladium acetate, palladium oxide, palladium chloride, palladium propionate, dichlorobis(triphenylphosphine) palladium, di-µ-chlorobis(η-allyl) palladium, dichloro(η-1,5-cylcooctadiene) palladium, dichloro(η-2,5-norbornadiene) palladium, dichlorobis(acetonitrile) palladium, dichlorobis(benzonitrile) palladium, dichlorobis(N,N-dimethylformamide) palladium, and bis(acetylacetonato) palladium.

The amount of such a palladium catalyst is usually 0.01 mol% or higher, relative to 1 mole of the linear sulfone compound (5). The upper limit is not particularly restricted, but the amounts not higher than 10 mol% are usually preferred from an economical point of view.

In the above reaction, a ligand may be used and the ligand may include phosphorous ligands such as optionally substituent(s)-containing triarylphosphines, trialkylphosphines, tris(dialkylamino)phosphines, diphosphine derivatives, triarylphosphites, and trialkylphosphites, specific examples of which are triphenylphosphine, tri-t-butylphosphine, tri-o-tolylphosphine, tri-m-tolylphosphine, tri-p-tolylphosphine, tris(dimethylamino)phosphine, 1,2-bis(diphenylphosphino)ethane, 1,3-bis(diphenylphosphino)propane, 1,4-bis(diphenylphosphino)butane, 1,2-bis(dimethylphosphino)ethane, 1,1'-bis(dimethylphosphino)ferrocene, 1,1'-bis(diphenylphosphino)ferrocene, triphenylphosphite, trimethylphosphite, tris(tridecyl)phosphite, tri-o-tolylphosphite, and tris(2,4-di-t-butylphenyl)phosphite. The amount of such a phosphorous ligand is usually in the range of 1 to 50 moles, preferably about 2 to 10 moles, relative to 1 mole of the palladium catalyst.

In the above reaction, an organic solvent is usually used. The solvent used may include ether solvents such as diethyl ether, tetrahydrofuran, 1,4-dioxane, dimethoxyethane, and anisole; alcohol solvents such as methanol, ethanol, 2-propanol, and t-butanol; aprotic polar solvents such as acetonitrile, N,N-dimethylformamide, hexamethylphosphoric triamide, sulfolane, 1,3-dimethyl-2-imidazolidinone, and 1-methyl-2-pyrrolidinone; and hydrocarbons solvents such as n-hexane, cyclohexane, n-pentane, benzene, toluene, and xylene. These may be used alone or as a mixture of two or more.

The reaction temperature can freely be selected in the range of -78°C to the boiling point of a solvent, preferably in the range of about 20°C to 60°C. After the reaction, the linear disulfone compound (3) can be obtained by the ordinary post-treatment, for examples, operations including water washing, extraction, crystallization, and various chromatographies.

The linear sulfone compound of formula (5) can be produced by reacting the sulfone compound of formula (6) with the allyl halide compound of formula (7) in the presence of a basic compound.

The basic compound used in the above reaction may include alkyl lithium, hydrides of alkali metal, hydroxides of alkali metals, alkoxides of alkali metals, and Grignard reagents, specific examples of which n-butyl lithium, s-butyl lithium, t-butyl lithium, sodium hydride, sodium hydroxide, potassium hydroxide, sodium methoxide, potassium methoxide, potassium t-butoxide, sodium t-butoxide, ethyl magnesium bromide, and ethyl magnesium chloride. The amount of such a basic compound used is usually about 0.5 to 3 moles, but about 1 to 20 moles for hydroxides of alkali metals, relative to 1 mole of the sulfone compound (6).

In the above reaction, a phase transfer catalyst may be used. The phase transfer catalyst may include the same as described above. In particular, quaternary ammonium salts are preferably used.

The amount of such a phase transfer catalyst used is usually about 0.01 to 0.2 mole, preferably about 0.02 to 0.1 mole, relative to 1 mole of sulfone compound (6).

In the above reaction, an organic solvent is usually used. The solvent used may include aprotic polar solvents such as acetonitrile, N,N-di-methylformamide, hexamethylphosphoric triamide, sulfolane, 1,3-dimethyl-2-imidazolidinone, and 1-methyl-2-pyrrolidinone; ether solvents such as diethyl ether, tetrahydrofuran, 1,4-dioxane, dimethoxyethane, and anisole; and hydrocarbon solvents such as n-hexane, cyclohexane, n-pentane, benzene, toluene, and xylene. These may be used alone or as a mixture of two or more. Preferably, N,N-dimethylformamide and tetrahydrofuran are used.

The reaction temperature can freely be selected in the range of-78°C to the boiling point of a solvent, preferably in the range of about -60°C to 40°C.

After the reaction, the linear sulfone compound (5) can be obtained by the ordinary post-treatment, for example, operations such as water washing, extraction, crystallization, and various chromatographies. Depending on the reaction conditions, an alcohol may be obtained at about 10% to 30% as the linear sulfone compound (5) wherein R is hydrogen, and can be reprotected according to the ordinary method (Greene, T.W., Protective Groups in Organic Synthesis, 3rd Edition, Wiley).

The linear disulfone compound of formula (3) can also be produced in one step by reacting the sulfone compound of formula (6) with the halosulfone compound of formula (8) in the presence of a basic compound.

The basic compound used in the above reaction may include the same as described above for use in the production of the linear sulfone compound (5). The amount of such a basic compound is usually about 0.5 to 3 moles, but about 1 to 20 moles for the hydroxide of an alkali metal, relative to 1 mole of the sulfone compound (6).

In the above reaction, a phase transfer catalyst may be used. The phase transfer catalyst may include the same as described above. In particular, quaternary ammonium salts are preferably used.

The amount of such a phase transfer catalyst used is usually about 0.01 to 0.2 mole, preferably about 0.02 to 0.1 mole, relative to 1 mole of the sulfone compound (6).

For the reaction, an organic solvent is usually used. The solvent used may include aprotic polar solvents such as acetonitrile, N,N-dimethyl-formamide, hexamethylphosphoric triamide, sulfolane, 1,3-dimethyl-2-imid-azolidinone, and 1-methyl-2-pyrrolidinone; ether solvents such as diethyl ether, tetrahydrofuran, 1,4-dioxane, dimethoxyethane, and anisole; and hydrocarbon solvents such as, n-hexane, cyclohexane, n-pentane, benzene, toluene, and xylene. These may be used alone or as a mixture of two or more. Preferably, N,N-dimethylformamide and tetrahydrofuran are used.

The reaction temperature can freely be selected in the range of-78°C to the boiling point of a solvent, preferably in the range of about -60°C to 40°C.

The cyclic disulfone compounds (4) and cyclic sulfone compounds (2) of the present invention can be led to retinol derivatives in a simple and easy manner according to the following schemes. More particularly, the cyclic disulfone compounds (4) are reacted with allyl halide compounds (7) to give coupling substances (12), which are then reacted with a base to give retinol derivatives. The cyclic sulfone compounds (2) are reacted with allyl halide compounds (7) in the same manner to give coupling substances (13), which are then reacted with a base to give retinol derivatives. 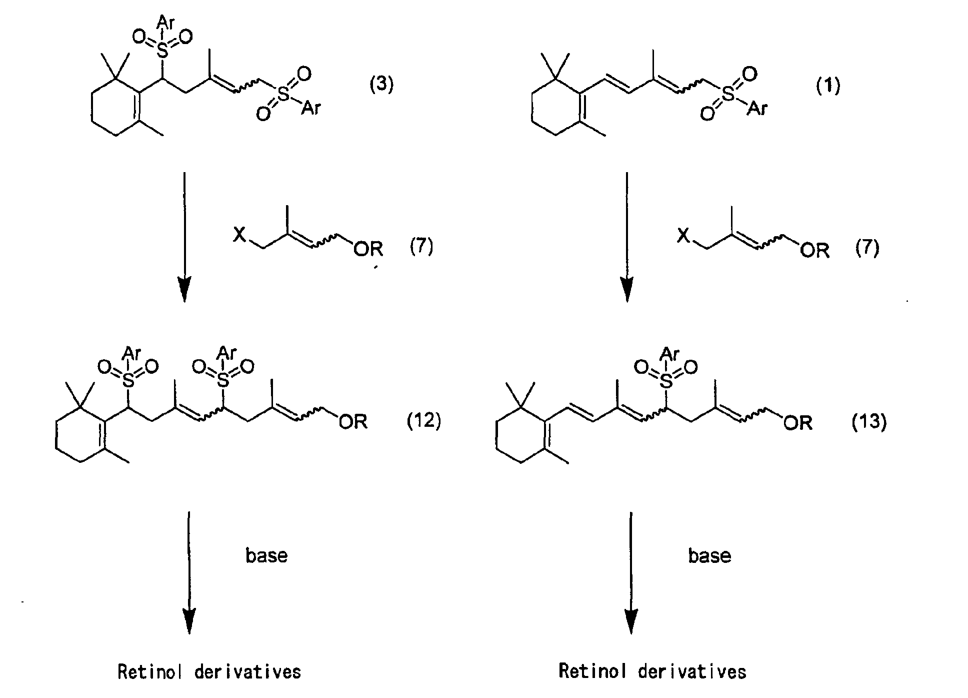 wherein X, R, Ar, and the wavy line are as defined above.

The present invention will hereinafter be further illustrated by the following examples; however, the present invention is not limited to these examples.

### Example 1

A solution of 12.5 g (130 mmol) of sodium t-butoxide dissolved in 150 ml of N,N-dimethylformamide (DMF) was cooled to 0°C, to which a solution of 29.2 g (100 mmol) of sulfone (VI) in DMF (50 ml) was added dropwise over 2 minutes. The reaction mixture was then cooled to -50°C, to which a solution of 22.8g (110 mmol) of allyl halide (VII) in DMF (100 ml) was added dropwise at the same temperature over 5 minutes, followed by stirring for 3 hours. After the reaction, the reaction mixture was poured into a saturated aqueous ammonium chloride solution, followed by extraction with ethyl acetate. The resulting organic layer was washed a saturated aqueous sodium hydrogencarbonate solution, a saturated aqueous sodium chloride solution in this order, dried over anhydrous magnesium sulfate, and then evaporated to remove the solvent, which attained a crude product. The resulting crude product was purified by silica gel column chromatography to give the desired sulfone (V) as a pale yellow oil in 50% yield.

### Example 2

First, 5.0 g (17.1 mmol) of sulfone (VI) was dissolved in 30 ml of tetrahydrofuran (THF), and this solution was cooled to -60°C, to which 12.2 ml (18.8 mmol) of 1.54 mol/l THF solution of n-butyl lithium was added dropwise over 5 minutes. The reaction mixture was stirred at the same temperature for 30 minutes, to which a solution of 3.6 g of allyl halide (VII) in THF (20 ml) was added dropwise at the same temperature over 5 minutes, followed by stirring for 3 hours. After the reaction, the reaction mixture was poured into a saturated aqueous ammonium chloride solution, followed by extraction with ethyl acetate. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was purified by silica gel column chromatography to give the desired sulfone (V) as a pale yellow oil in 41% yield.

### Example 3

A solution of 56 mg (1.4 mmol) of sodium hydride in DMF (5 ml) was cooled to 0°C, to which a solution of 292 mg (1 mmol) of sulfone (VI) in DMF (3 ml) was added dropwise at the same temperature. After dropwise addition, the reaction mixture was stirred at 5°C to 10°C for 20 minutes. A solution of 260 mg (1.2 mmol) of allyl halide (VII) in DMF (3 ml) was then added dropwise at the same temperature, followed by stirring for 5 hours. After the reaction, the reaction mixture was poured into a saturated aqueous ammonium chloride solution, followed by extraction with ethyl acetate. The resulting organic layer was washed with a saturated aqueous sodium chloride solution, dried over anhydrous magnesium, and then evaporated to remove the solvent, which afforded a crude product. The resulting crude product was subjected to quantitative analysis by liquid chromatography, and it was found that the yield of sulfone (V) was 77%.

### Example 4

To THF (1.5 ml) were added 842 g (15 mmol) of potassium hydroxide and 32 mg (0.1 mmol) of tetrabutylammonium bromide, and while the mixture was stirred at room temperature, a solution of 324 mg (1.5 mmol) of allyl halide (VII) and 292 mg (1 mmol) of sulfone (VI) in THF (1 ml) was added dropwise at the same temperature, followed by stirring for 30 minutes. After the reaction, the reaction mixture was poured into a saturated aqueous ammonium chloride solution, followed by extraction with ethyl acetate. The resulting organic layer was washed with a saturated aqueous sodium chloride solution, dried over anhydrous sodium sulfate, and then evaporated to remove the solvent, which afforded a crude product. The resulting crude product was purified by silica gel column chromatography to give the desired sulfone (V) as a pale yellow oil in 93% yield.

### Example 5

The reaction was carried out according to Example 4, except that 601 mg (15 mmol) of sodium hydroxide was used instead of potassium hydroxide. The resulting crude product was subjected to quantitative analysis by liquid chromatography, and it was found that the yield of sulfone (V) was 71%.

### Example 6

The reaction was carried out according to Example 4, except that 253 mg (1.5 mmol) of allyl halide (IX) was used instead of allyl halide (VII). The resulting crude product was subjected to quantitative analysis by liquid chromatography, and it was found that the yield of sulfone (V) was 82%.

### Example 7

First, 4.19 mg (10 mmol) was dissolved in a mixed solvent of 20 ml of methanol and 20 ml of tetrahydrofuran, to which 3.56 g (20 mmol) of anhydrous sodium p-toluenesulfinate, 225 mg (1 mmol) of palladium acetate, and 621 mg (2 mmol) triphenylphosphite were added. The reaction mixture was then stirred at room temperature for 24 hours. After completion of the reaction, water was poured into the reaction mixture, followed by extraction with ethyl acetate.

The resulting organic layer was washed with a saturated aqueous sodium chloride solution, dried over anhydrous magnesium sulfate, and then evaporated to remove the solvent, which afforded a crude product. The resulting crude product was purified by silica gel column chromatography to give sulfone (III) as white crystals in 70% yield.

The sulfone (III) was obtained as a mixture of trans and cis forms (trans/cis = 85/15).
Sulfone (III), trans form
Rf= 0.13 (n-hexane/ethyl acetate = 3/1)
¹H-NMR δ (CDCl₃):
1.19 (3H, s), 1.35 (3H, s), 1.58 (3H, s), 1.67 (3H, s), 1.95 (4H, s), 2.26-2.30 (1H, m), 2.44 (6H, s), 2.88-2.92 (1H, m), 3.73 (2H, d, J = 8 Hz), 3.80-3.84 (1H, m), 4.85 (1H, d, J = 10 Hz), 5.01 (1H, s), 5.17 (1H, t, J = 8 Hz), 7.29-7.39 (4H, m), 7.68-7.82 (4H, m)
Sulfone (III), cis form
Rf = 0.18 (n-hexane/ethyl acetate = 3/1)
¹H-NMR δ (CDCl₃):
1.16 (3H, s), 1.58 (3H, s), 1.67 (3H, s), 1.71 (3H, s), 1.92 (4H, s), 2.05-2.13 (1H, m), 2.44 (3H, s), 2.47 (3H, s), 2.75-2.79 (1H, m), 3.65-3.71 (1H, m), 3.90-4.02 (2H, m), 4.89 (1H, d, J = 10 Hz), 4.98 (1H, s), 5.34 (1H, t, J = 8 Hz), 7.20-7.38 (4H, m), 7.69-7.82 (4H, m)

### Example 8

A solution of 112 mg (1 mmol) of sodium t-butoxide dissolved in 3 ml of N,N-dimethylformamide (DMF) was cooled to -20°C, to which a solution of 295 mg (1 mmol) of sulfone (VI) in DMF (1.5 ml) was added dropwise for 2 minutes. The reaction mixture was cooled to -60°C, to which a solution of 175 mg (0.5 mmol) of halosulfone (VIII) in DMF (1.5 ml) was added dropwise at the same temperature over 5 minutes, followed by stirring at the same temperature for 3 hours. After the reaction, the reaction mixture was poured into a saturated aqueous ammonium chloride solution, followed by extraction with ethyl acetate. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was purified by silica gel column chromatography to give the desired sulfone (III) as a pale yellow oil in 22% yield.

### Example 9

First, 4.82 g (9 mmol) of sulfone (III) was dissolved in 90 ml of toluene, which was charged with 7.57 g (135 mmol) of 99% potassium hydroxide, 577 mg (18 mmol) of methanol, and 103 mg (0.45 mmol) of benzyltriethylammonium chloride, followed by stirring at 45°C for 1.5 hours. After the reaction, the reaction mixture was poured into a saturated aqueous ammonium chloride solution, followed by extraction with ethyl acetate. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was purified by silica gel column chromatography to give sulfone (I) as a pale yellow solid in 53% yield.
Sulfone (I)
Rf = 0.37 (n-hexane/ethyl acetate = 3/1)
¹H-NMR δ (CDCl₃):
1.49 (3H, s), 1.61 (3H, s), 1.69 (3H, s), 1.76 (3H, s), 2.10 (4H, s), 2.44 (3H, s), 3.93 (2H, d, J = 8 Hz), 5.07-5.10 (1H, m), 5.38 (1H, t, J = 8 Hz), 5.70-5.73 (1H, m), 5.87-5.96 (1H, m), 6.37-6.46 (1H, m), 7.32 (2H, d, J = 8 Hz), 7.73 (2H, d, J = 8 Hz)

### Example 10

A mixed solution of 240 mg (2.2 mmol) of sulfuric acid and 95 mg (1.6 mmol) of acetic acid was cooled to 0°C, to which 106 mg (0.2 mmol) of sulfone (III) was added in eight portions over 40 minutes. The reaction mixture was heated to 15°C and stirred for 10 minutes. After the reaction, ice water was poured into the reaction mixture, followed by extraction with ethyl acetate. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was purified by silica gel column chromatography to give the desired sulfone (IV) as a pale yellow oil in 8% yield.

### Example 11

To 106 mg (0.2 mmol) of sulfone (III) and 9 mg (0.04 mmol) of benzyltriethylammonium chloride was added 4 ml of toluene, and to this mixture was added 8 ml of 60% perchloric acid at room temperature. The reaction mixture was then stirred at room temperature for 28 hours. After the reaction, water was poured, and the mixture was extracted with ethyl acetate. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was analyzed by high performance liquid chromatography, and it was found that the yield of sulfone (IV) was 32%.

### Example 12

First, 75 mg (0.2 mmol) of sulfone (I) was dissolved in 1 ml of acetic acid, and to this solution was added 95 mg (0.7 mmol) of zinc chloride at room temperature. The reaction mixture was then heated to 40°C and stirred for 2 hours. After completion of the reaction, the reaction mixture was poured into a saturated sodium hydrogencarbonate solution, followed by extraction with ethyl acetate. The resulting organic layer was washed with a saturated aqueous solution chloride solution, dried over anhydrous magnesium sulfate, and then evaporated to remove the solvent, which afforded a crude product. The resulting crude product was analyzed by high performance liquid chromatography, and it was found that the yield of sulfone (II) was 32%.

### Example 13

The reaction was carried out according to Example 12, except that zinc bromide was used instead of zinc chloride and acetic acid containing 2,6-di-t-butyl-p-cresol at 300 ppm was used as a solvent, to give sulfone (II) in 24% yield.

### Example 14

First, 75 mg (0.2 mmol) of sulfone (I) was dissolved in 1 ml of toluene containing 2,6-di-t-butyl-p-cresol at 300 ppm, and to this solution was added 30 mg (40 wt%) of sulfated zirconia at room temperature, followed by heating to 50°C and stirring for 2.5 hours. After completion of the reaction, the reaction mixture was filtered through celite and evaporated to remove the solvent, which afforded a crude product. The resulting crude product was analyzed by high performance liquid chromatography, and it was found that the yield of sulfone (II) was 20%.

### Example 15

First, 75 mg (0.2 mmol) of sulfone (I) was dissolved in 1 ml of toluene containing 2,6-di-t-butyl-p-cresol at 300 ppm, and to this solution was added 50 mg (67 wt%) of Amberlist 15DRY at room temperature, followed by heating to 40°C and stirring for 1 hour. After completion of the reaction, water was added to the reaction mixture, and after phase separation, the organic layer was further washed twice with water. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was analyzed by high performance chromatography, and it was found that the yield of sulfone (II) was 29%.

### Example 16

First, 75 mg (0.2 mmol) of sulfone (I) was dissolved in 1 ml of dichloromethane, and to this solution was added 50 mg (67 wt%) of Amberlist 15DRY at room temperature, followed by heating to 40°C and stirring for 1 hour. After completion of the reaction, water was added, and after phase separation, the organic layer was further washed twice with water. The resulting organic layer was washed with a saturated aqueous sodium hydrogencarbonate solution, a saturated aqueous sodium chloride solution in this order, dried over anhydrous magnesium sulfate, and then evaporated to remove the solvent, which afforded a crude product. The resulting crude product was analyzed by high performance liquid chromatography, and it was found that the yield of sulfone (II) was 55%.
The following will show the structural formulas of the compounds of Examples, in which Ts is p-tolylsulfonyl.

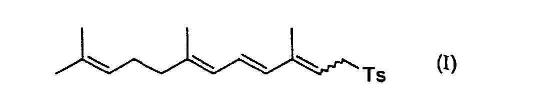

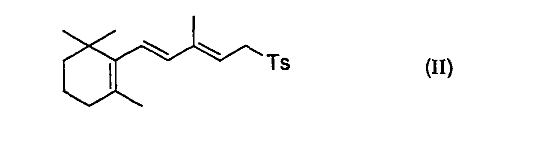

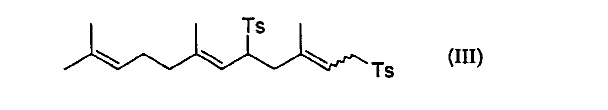

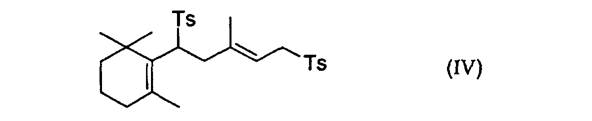

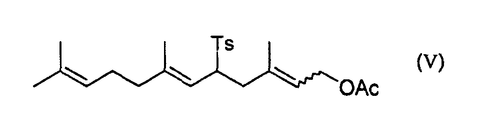

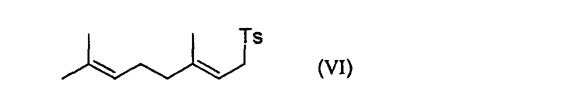

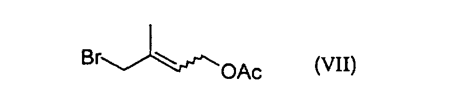

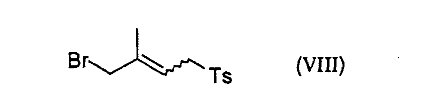

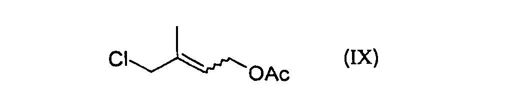

### Industrial Applicability

According to the present invention, cyclic sulfone derivatives useful as the intermediates of retinol derivatives or carotenoids can be produced using inexpensive isoprene in a simple and easy manner.

## Claims

1. A process for the production of a cyclic sulfone compound of formula (2): 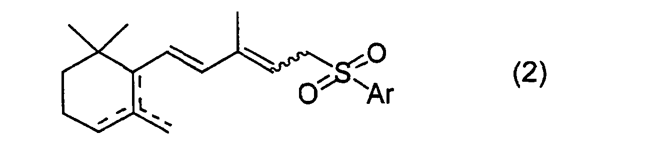 wherein the dashed lines represent that a double bond is present in one of three positions indicated; Ar and the wavy line are as defined below, **characterized in that** a conjugated triene compound of formula (1): 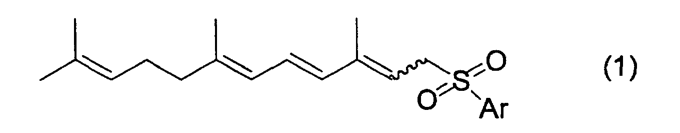 wherein Ar is aryl optionally having a substituent(s) and the wavy line represents either one of E/Z geometrical isomers or their mixture, is subjected to cyclization in the presence of an acid catalyst.

2. A process for the production of a conjugated triene derivative of formula (1): 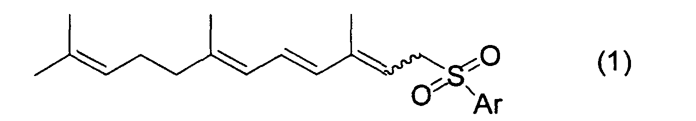 wherein Ar and the wavy line are as defined below, **characterized in that** a linear disulfone compound of formula (3): 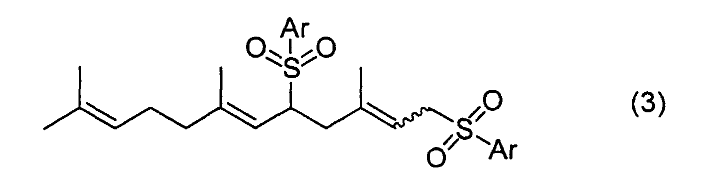 wherein Ar is aryl optionally having a substituent(s) and the wavy line represents either one of E/Z geometrical isomers or their mixture, is reacted with a base.

3. A process for the production of a cyclic disulfone compound of formula (4): 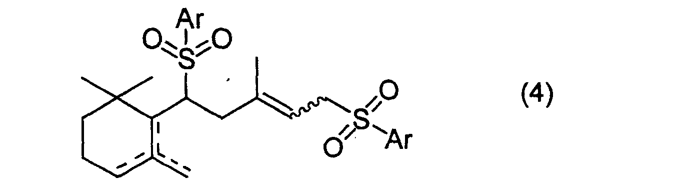 wherein Ar, the dashed lines, and the wavy line are as defined below, **characterized in that** a linear disulfone compound of formula (3): 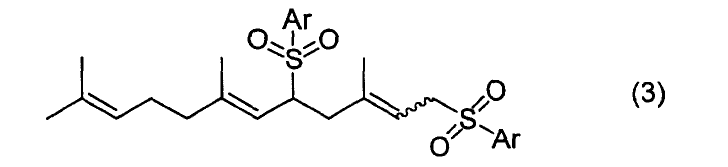 wherein Ar is aryl optionally having a substituent(s) and the wavy line represents either one of E/Z geometrical isomers or their mixture, is subjected to cyclization in the presence of an acid catalyst.

4. A process of the production of a linear disulfone compound of formula (3): 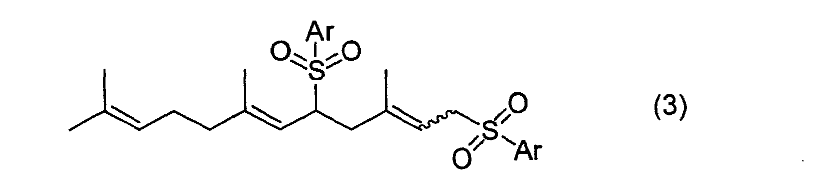 wherein Ar and the wavy line are as defined below, **characterized in that** a linear sulfone compound of formula (5): 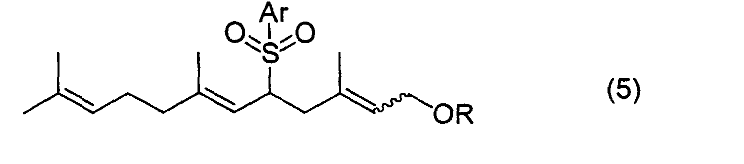 wherein Ar is aryl optionally having a substituent(s), the wavy line represents either one of E/Z geometrical isomers or their mixture, and R is a protective group for the hydroxyl group, is reacted with an arylsulfinate of formula (9):
ArSO₂M (9)
wherein Ar is as defined above and M is an alkali metal.

5. A process for the production of a linear disulfone compound of formula (3): 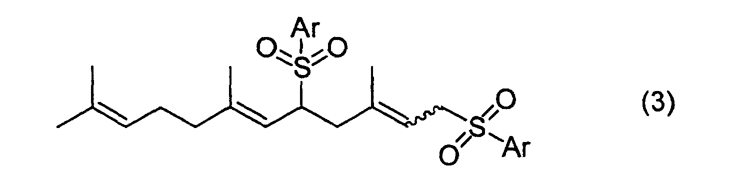 wherein Ar and the wavy line are as defined below, **characterized in that** a sulfone compound of formula (6): 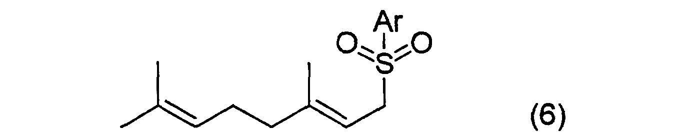 wherein Ar is aryl optionally having a substituent(s), is reacted with an allyl halide of formula (7): 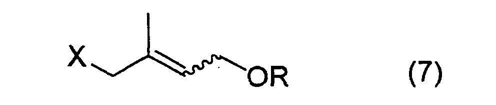 wherein X is halogen; R and the wavy line are as defined below, in the presence of a basic compound to give a linear sulfone compound of formula (5): 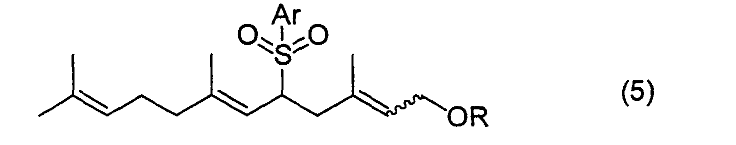 wherein Ar and the wavy line are as defined above, and the resulting linear sulfone (5) is reacted with an arylsulfinate of formula (9):
ArSO₂M (9)
wherein Ar is as defined above and M is an alkali metal.

6. A process for producing a linear disulfone compound of formula (3): 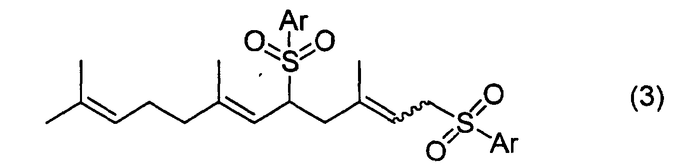 wherein Ar and the wavy line are as defined below, **characterized in that** a sulfone compound of formula (6): 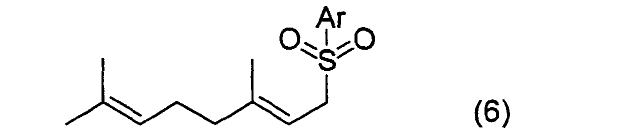 wherein Ar is aryl optionally having a substituent(s), is reacted with a halosulfone compound of formula (8): 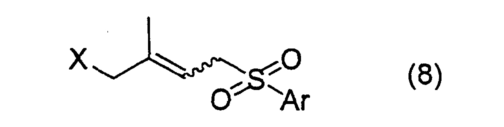 wherein X is halogen; Ar and the wavy line are as defined above, in the presence of a basic compound.

7. The production process according to claim 1 or 3, wherein the acid catalyst is a protic acid.

8. The production process according to claim 7, wherein the protic acid is sulfuric acid, acetic acid, hydrochloric acid, phosphoric acid, trifluoroacetic acid, perchloric acid, perbromic acid, periodic acid, benzenesulfonic acid, p-toluenesulfonic acid, methanesulfonic acid, or trifluoromethanesulfonic acid.

9. The production process according to claim 1 or 3, wherein the acid catalyst is a Lewis acid.

10. The production process according to claim 9, wherein the Lewis acid is a halide of zinc, aluminum, zirconium, tin, copper, titanium, or boron.

11. The production process according to claim 1 or 3, wherein the acid catalyst is a solid acid.

12. The production process according to claim 11, wherein the solid acid is zeolite, a cation exchange resin, or a sulfuric acid treated product of zirconia, titania, or alumina.

13. The production process according to claim 12, wherein the functional groups of the cation exchange resin are sulfonic acid groups.

14. The production process according to claim 2, wherein the base is a hydroxide of an alkali metal, a hydride of an alkali metal, an alkoxide of an alkali metal, or an amide of an alkali metal.

15. The production process according to claim 14, wherein the hydroxide of an alkali metal is potassium hydroxide or sodium hydroxide.

16. The production process according to claim 2, **characterized in that** the linear disulfone compound of formula (3) is reacted with a base in the presence of a phase transfer catalyst and a lower alcohol to give the conjugated triene compound of formula (1).

17. The production process according to claim 16, wherein the phase transfer catalyst is a quaternary ammonium salt.

18. The production process according to claim 16, wherein the lower alcohol is methanol, ethanol, isopropyl alcohol, or ethylene glycol.

19. The production process according to claim 19, wherein the sulfonation is carried out using an arylsulfinate of formula (9) in the presence of a palladium catalyst.

20. The production process according to claim 19, wherein the sulfonation is carried out in the presence of a palladium catalyst and a phosphorous ligand.

21. The production process according to claim 19, wherein the arylsulfinate is sodium p-toluenesulfinate or potassium p-toluenesulfinate.

22. The production process according to claim 5 or 6, wherein the basic compound is an alkyl lithium, an alkali metal hydride, a hydroxide of an alkali metal, an alkoxide of an alkali metal, or a Grignard reagent.

23. The production process according to claim 5 or 6, wherein when the basic compound is a hydroxide of an alkali metal, a phase transfer catalyst is added.

24. The production process according to claim 23, wherein the phase transfer catalyst is a quaternary ammonium salt.

25. The production process according to claim 23, wherein the alkali metal is sodium or potassium.

26. The production process according to claim 22, wherein the alkoxide of an alkali metal is sodium t-butoxide or potassium t-butoxide.

27. The production process according to claim 4 or 5, wherein R is acyl.

28. The production process according to claim 4 or 5, wherein R is acetyl.

29. A conjugated triene compound of formula (10): 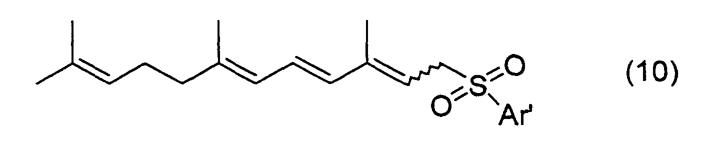 wherein Ar' is phenyl having a substituent(s), the wavy line represents either one of E/Z geometrical isomers or their mixture, and R is a protective group of the hydroxyl group.

30. A linear disulfone compound of formula (11): 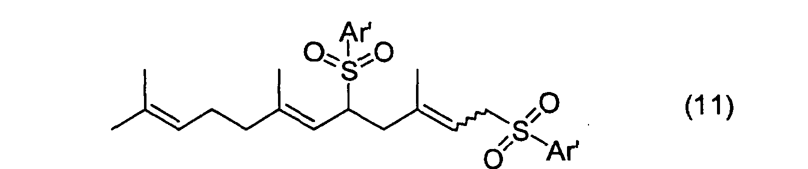 wherein Ar' is phenyl having a substituent(s), the wavy line represents either one of E/Z geometrical isomers or their mixture, and R is a protective group of the hydroxyl group.

31. The conjugated triene compound of formula (10) according to claim 29, wherein Ar' is p-tolyl.

32. The linear disulfone compound of formula (11) according to claim 30, wherein Ar' is p-tolyl.
